# FASCICULE DE BREVET EUROPEEN

(11) **EP 3 761 930 B1**
(45) Date de publication et mention de la délivrance du brevet: **11.01.2023**
(21) Numéro de dépôt: 19723153.3
(22) Date de dépôt: 12.04.2019
(51) Int. Cl.: G02C 3/02, A61F 9/02, G21F 3/02, G02C 5/14, G02C 5/00, G02C 11/00

(54) **MONTURE DE LUNETTES À PROTECTION AMÉLIORÉE CONTRE LES RAYONNEMENTS IONISANTS ET LUNETTES DE RADIOPROTECTION COMPRENANT UNE TELLE MONTURE**
BRILLENRAHMEN MIT ERHÖHTEM STRAHLUNGSSCHUTZ VOR IONISIERENDER STRAHLUNG UND BRILLE UMFASSEND EINEN SOLCHEN RAHMEN
IMPROVED PROTECTIVE EYEGLASS FRAMES AGAINST IONIZING RADIATION AND RADIOAPROTECTION EYEGLASSES COMPRISING SUCH FRAME

(30) Priorité: 16.04.2018 FR 1853320
(43) Date de publication de la demande: 13.01.2021
(73) Titulaire: Orano Recyclage, 92320 Châtillon (FR)
(72) Inventeur: JEHANNO, Jacky, 30330 Gaujac (FR)
(74) Mandataire: Brevalex
(86) Numéro de dépôt international: PCT/FR2019/050868
(87) Numéro de publication internationale: WO 2019/202243

(56) Documents cités:
- EP-A1- 3 348 244
- WO-A2-00/28551
- DE-A1- 3 616 253
- DE-U1- 8 532 493
- DE-U1- 8 910 235

## Description

### DOMAINE TECHNIQUE

L'invention se rapporte au domaine de la protection individuelle contre les rayonnements ionisants et, en particulier, contre les rayonnements électromagnétiques de type X et gamma.

Plus spécifiquement, elle se rapporte à une monture de lunettes à protection améliorée contre les rayonnements ionisants ainsi qu'à des lunettes de protection contre les rayonnements ionisants comprenant une telle monture.

Ces lunettes seront dénommées plus simplement « lunettes de radioprotection » dans ce qui suit.

L'invention se rapporte également à un ensemble comprenant des lunettes de radioprotection et un dispositif de reprise du poids de ces lunettes.

L'invention trouve application dans tous les domaines dans lesquels des personnes sont susceptibles d'être exposées à des rayonnements ionisants dans l'exercice de leur activité professionnelle et, notamment, dans les domaines de la médecine (radiologie diagnostique, radiologie interventionnelle, radiothérapie, curiethérapie, médecine nucléaire, etc), de la recherche fondamentale et appliquée, et de l'industrie nucléaire (activités minières, conversion de l'uranium, traitement de combustibles nucléaires usés, fabrication de combustibles nucléaires neufs, etc).

### ÉTAT DE LA TECHNIQUE ANTÉRIEURE

La Directive 2013/59/Euratom du Conseil du 5 décembre 2013 fixant les normes de base relatives à la protection sanitaire contre les dangers résultant de l'exposition aux rayonnements ionisants a abaissé la limite de dose équivalente au cristallin pour les travailleurs. Elle impose désormais, dans des situations d'exposition planifiées liées à l'exercice d'une activité professionnelle, une limite de dose équivalente au cristallin de 20 millisieverts (mSv) par an, en moyenne sur des périodes définies de 5 ans, sans dépasser 50 mSv sur une même année, alors que cette limite était jusqu'alors de 150 mSv par an.

Le respect de cette Directive passe par le suivi dosimétrique approprié des travailleurs et/ou par le port systématique d'équipements individuels de radioprotection oculaire qui doivent non seulement protéger efficacement le cristallin mais également répondre à des critères d'ergonomie pour que les travailleurs se plient facilement à l'obligation de porter ces équipements.

Il existe trois types d'équipements individuels de radioprotection oculaire : les lunettes qui peuvent être à verres correcteurs ou non, les sur-lunettes qui sont destinées aux personnes porteuses de lunettes correctrices et les visières (aussi appelées « casques visières ») qui peuvent être portées aussi bien avec que sans lunettes correctrices et qui sont plutôt dédiées au domaine médical car elles assurent aussi une protection contre les projections de liquides biologiques tels que le sang.

De ces trois types d'équipements, les lunettes sont de loin les plus simples d'utilisation et ceux qui sont susceptibles d'être portés le plus facilement, fréquemment, voire systématiquement, par les travailleurs.

Les lunettes de radioprotection qui sont actuellement disponibles commercialement sont conçues pour fournir soit une protection exclusivement frontale par la présence de verres plombés soit une protection frontale doublée d'une protection latérale, auquel cas ces lunettes comprennent, outre des verres plombés, des éléments de protection latérale de taille limitée (typiquement de l'ordre de 2 cm de haut sur 1,5 cm de large) qui sont rapportés sur des retours latéraux que comprend la monture ou insérés dans ces retours latéraux.

Dans les deux cas, la monture est en un matériau plastique transparent aux rayonnements ionisants.

Les lunettes de radioprotection à protections frontale et latérale atténuent efficacement les rayonnements ionisants qui frappent les verres plombés et les éléments de protection latérale.

Par contre, elles n'atténuent ni les rayonnements ionisants qui frappent la monture ni ceux qui sont issus de l'interaction de rayonnements ionisants avec les zones cutanées recouvertes par la monture ou entourant cette monture. Or, selon leur direction de propagation, ces rayonnements ionisants, qui ne sont pas atténués par les lunettes, sont aussi susceptibles d'atteindre le cristallin.

EP-A-3 348 244, qui est état de la technique uniquement au titre de l'Article 54(3) CBE, décrit une monture de lunettes qui comporte une partie frontale monobloc avec deux éléments de protection latérale et dans laquelle la partie frontale et les éléments de protection latérale sont en un matériau constitué d'un mélange d'une matière plastique et d'un métal, ce métal étant le plomb ou le tungstène.

L'inventeur s'est donc fixé pour but de fournir des lunettes de radioprotection qui offrent une protection améliorée du cristallin contre les rayonnements ionisants.

Il s'est également fixé pour but que ces lunettes présentent de plus une ergonomie et, en particulier, un confort de port ainsi qu'une esthétique propres à favoriser leur utilisation systématique par des travailleurs qui sont soumis à une exposition à des rayonnements ionisants.

Il s'est aussi fixé pour but que les coûts de revient de ces lunettes permettent leur large diffusion dans les milieux professionnels dans lesquels une protection individuelle oculaire contre les rayonnements ionisants est recherchée.

### EXPOSÉ DE L'INVENTION

Ces buts sont atteints par l'invention qui propose en premier lieu une monture de lunettes de protection contre les rayonnements ionisants, qui comprend une partie frontale, ou face, se prolongeant latéralement par deux éléments de protection latérale, ainsi qu'un dispositif de maintien de la monture sur le visage d'un utilisateur en condition de port, et qui est caractérisée en ce que :
- les éléments de protection latérale sont monoblocs avec la partie frontale,
- la partie frontale et les éléments de protection latérale sont en un matériau radio-atténuateur comprenant une matrice polymère dans laquelle est dispersée une charge radio-atténuatrice, et en ce que
- la charge radio-atténuatrice comprend des particules d'au moins un métal choisi parmi les terres rares, le bismuth, l'antimoine, l'étain, le baryum, le tantale, ou d'au moins un composé choisi parmi les alliages et les oxydes de plomb, de terres rares, de bismuth, d'antimoine, d'étain, de tungstène, de baryum, de tantale.

Dans ce qui précède et ce qui suit, on entend :
- par « charge radio-atténuatrice », une matière qui se présente sous une forme divisée, plus précisément sous la forme de particules, et qui est capable d'interagir avec les photons issus d'un rayonnement ionisant et d'absorber une partie de l'énergie de ces photons ; et
- par « matériau radio-atténuateur », un matériau qui, parce qu'il comprend une charge radio-atténuatrice, est également capable d'interagir avec les photons issus d'un rayonnement ionisant et d'absorber une partie de l'énergie de ces photons.

Le rayonnement ionisant peut notamment être un rayonnement électromagnétique de type X si celui-ci est produit par un générateur de rayons X au sein duquel une différence de potentiel allant classiquement de plusieurs dizaines à plusieurs centaines de kilovolts (kV) est appliquée, ou un rayonnement électromagnétique de type gamma si celui-ci est émis par un ou plusieurs atomes radioactifs lors de leur dési ntégration.

Par ailleurs, on entend par « polymère », aussi bien un homopolymère, c'est-à-dire un polymère qui est issu de la polymérisation d'une seule espèce de monomères, qu'un copolymère, c'est-à-dire un polymère qui est issu de la copolymérisation de plusieurs espèces de monomères.

Comme précédemment indiqué, la charge radio-atténuatrice comprend des particules d'au moins un métal choisi parmi les terres rares, le bismuth, l'antimoine, l'étain, le baryum, le tantale, ou d'au moins un composé choisi parmi les alliages et les oxydes de plomb, de terres rares, de bismuth, d'antimoine, d'étain, de tungstène, de baryum, de tantale.

On rappelle que les terres rares sont un groupe de métaux comprenant le scandium (Sc), l'yttrium (Y) et l'ensemble des lanthanides, ces derniers correspondant aux 15 éléments chimiques répertoriés dans le tableau périodique des éléments de Mendeleïev allant du numéro atomique 57 pour le lanthane (La) au numéro atomique 71 pour le lutécium (Lu).

Ainsi, la charge atténuatrice peut notamment comprendre des - ou être composée de - particules :
- d'un oxyde de plomb et, en particulier, de monoxyde de plomb de formule PbO,
- d'erbium ou d'un oxyde d'erbium et, en particulier, de sesquioxyde d'erbium(III) de formule Er₂O₃,
- de praséodyme ou d'un oxyde de praséodyme et, en particulier, d'oxyde de praséodyme(III-IV) de formule Pr₆O₁₁;
- de bismuth ou d'un oxyde de bismuth tel que le sesquioxyde de bismuth de formule Bi₂O₃ ;
- d'un mélange d'un oxyde d'erbium et d'un oxyde de praséodyme tel qu'un mélange d'Er₂O₃ et de Pr₆O₁₁, par exemple dans des proportions massiques de 55% à 65% d'Er₂O₃ et de 35% à 45% de Pr₆O₁₁, rapportées à la masse du mélange,
- d'un mélange d'un oxyde d'erbium, d'un oxyde de praséodyme et d'un oxyde de bismuth tel qu'un mélange d'Er₂O₃, de Pr₆O₁₁ et de Bi₂O₃, par exemple dans des proportions massiques de 30% à 45% d'Er₂O₃, 20% à 30% de Pr₆O₁₁ et 30% à 45% de Bi₂O₃, rapportées à la masse du mélange, ou encore
- d'un mélange d'un oxyde de bismuth, d'un oxyde de tungstène et d'un oxyde de lanthane tel qu'un mélange de Bi₂O₃, de trioxyde de tungstène de formule WO₃ et de trioxyde de lanthane de formule La₂O₃, par exemple dans des proportions massiques de 70% à 90% en masse de Bi₂O₃, 5% à 15% en masse de WO₃ et de 5% à 15% de La₂O₃, rapportées à la masse du mélange.

La charge radio-atténuatrice est préférentiellement choisie en fonction de l'énergie photonique des rayonnements ionisants destinés à être atténués par les lunettes de radioprotection et, donc, par leur monture.

Ainsi, dans les installations de fabrication de combustible nucléaire de type MOX (pour « **M**ixed **OX**ide ») mettant en œuvre des poudres d'oxydes d'uranium et de plutonium, les rayonnements majoritaires étant des rayonnements X issus des radio-isotopes du plutonium notamment, de l'américium et de l'uranium 237 et correspondant à des rayonnements ionisants d'énergie photonique respective de 60 keV et 208 keV, on choisira par exemple une charge radio-atténuatrice à base de bismuth ou d'oxyde de bismuth.

Le polymère de la matrice est choisi en fonction des qualités que l'on souhaite conférer à la monture, en particulier en termes de souplesse, de flexibilité, de poids, de solidité (résistance aux chocs, résistance aux agents chimiques, etc) et d'esthétique (couleurs, textures, etc), ainsi que du coût de revient de cette monture.

Ainsi, il peut notamment s'agir d'un acétate de cellulose tel que ceux commercialisés par MAZZUCHELLI et PLASTIMOD, d'un acétopropionate de cellulose tel que ceux commercialisés par EASTMAN Chemical Company sous les références Tenite^{™}, d'un polyamide ou copolyamide tel que ceux commercialisés par EMS-Grivory sous les références Grilamid^{™} ou ceux commercialisés par ARKEMA sous les références Rielsan^{™}, d'une résine époxyde telle que l'Optyl^{™}, ou encore d'un polyuréthanne tel que ceux commercialisés par COVESTRO sous la référence Desmopan^{™}.

Parmi ceux-ci, préférence est donnée :
- d'une part, aux polyamides et copolyamides et, notamment, au Grilamid^{™} TR 90 d'EMS-Evory en raison de son extrême légèreté (ce qui est une qualité très appréciable pour la réalisation de montures enveloppantes), de sa résilience (puisqu'il s'agit d'un polymère à mémoire de forme) et de ses propriétés exceptionnelles de résistance, en particulier aux chocs, et
- d'autre part, aux polyuréthannes.

Conformément à l'invention, la proportion massique de la charge radio-atténuatrice dans le matériau radio-atténuateur et/ou l'épaisseur de ce matériau sont préférentiellement choisies de sorte que ce matériau présente une équivalence plomb au moins égale à 0,4 mm, c'est-à-dire qu'il assure une protection contre les rayonnements ionisants équivalente à celle conférée par une plaque de plomb de 0,4 mm d'épaisseur.

Cette proportion massique, qui varie en fonction de la composition de la charge radio-atténuatrice utilisée, de l'épaisseur du matériau radio-atténuateur ainsi que du niveau d'atténuation des rayonnements ionisants recherché et qui est donc à adapter au cas par cas - ce que saura faire un homme du métier - est typiquement comprise entre 20% et 95% rapportés à la masse du matériau radio-atténuateur.

La monture de l'invention peut présenter des formes très différentes en fonction de l'usage auquel sont destinées les lunettes de radioprotection et, notamment, de la surface de protection recherchée dans le cadre de cet usage mais aussi en fonction des critères ergonomiques et esthétiques auxquels on souhaite que ces lunettes répondent.

En premier lieu, il peut s'agir d'une monture dont la partie frontale est configurée pour enchâsser deux verres optiques qui sont destinés à couvrir chacun l'une des deux zones orbitaires d'un utilisateur en condition de port.

En variante, il peut s'agir d'une monture dite « mono-écran », c'est-à-dire dont la partie frontale est configurée pour n'enchâsser qu'un seul verre optique qui est destiné à couvrir les deux zones orbitaires d'un utilisateur en condition de port.

Dans un cas comme dans l'autre, la monture peut avoir une forme plus ou moins couvrante, voire enveloppante (dite « wrap-around » en anglais) selon la surface de protection recherchée.

Ainsi, il peut s'agir d'une monture dont la partie frontale est plane ou à faible degré de courbure, c'est-à-dire avec un angle de cintre compris entre 0° et 10°.

En variante, il peut s'agir d'une monture dont la partie frontale présente un degré de courbure prononcé pour épouser davantage la forme du visage, c'est-à-dire avec un angle de cintre supérieur à 10° et pouvant aller jusqu'à 30°.

Par ailleurs, il peut s'agir d'une monture dans laquelle seuls participent à assurer une radioprotection la partie frontale de cette monture et les éléments de protection latérale.

En variante, il peut s'agir d'une monture qui comprend de plus deux éléments de protection sus-orbitaire et/ou deux éléments de protection sub-orbitaire, ces éléments de protection sus-orbitaire et/ou sub-orbitaire étant monoblocs avec la partie frontale et les éléments de protection latérale.

Le dispositif de maintien de la monture sur le visage d'un utilisateur en condition de port peut comprendre deux branches articulées sur les éléments de protection latérale, auquel cas ces branches peuvent être soit en matériau radio-atténuateur comme le reste de la monture soit être constituées d'un polymère exempt de charge radio-atténuatrice, ce polymère pouvant être le même que celui qui forme la matrice du matériau radio-atténuateur ou différente de celui-ci.

Pour des raisons d'ergonomie ou d'esthétique, les branches peuvent aussi être « bi-matériau », c'est-à-dire constituées en partie d'un premier matériau et en partie d'un deuxième matériau ayant des propriétés, par exemple de flexibilité, de poids, de solidité ou d'apparence, différentes de celles du premier matériau.

Par ailleurs, les branches peuvent être rectilignes avec, toutefois, une courbure à leurs extrémités pour assurer à un utilisateur un confort et une stabilité, ou bien être galbées de manière à épouser la tête d'un utilisateur. Dans tous les cas, leur longueur peut être réglable.

En variante, le dispositif de maintien de la monture sur le visage d'un utilisateur en condition de port peut comprendre un bandeau relié, de façon détachable ou non, aux éléments de protection latérale, auquel cas ce bandeau est avantageusement en une matière élastique, par exemple un textile élastique ou un élastomère tel que le néoprène, et ajustable à la circonférence de la tête d'un utilisateur.

Conformément à l'invention, la partie frontale de la monture présente, de préférence, une hauteur comprise entre 3,5 cm et 5,5 cm et une largeur comprise entre 13 cm et 17 cm.

Quant aux éléments de protection latérale, ils présentent, de préférence, une longueur au moins égale à 2 cm et, mieux encore, comprise entre 2,5 cm et 3,5 cm, et une hauteur qui, en tout point de leur longueur, est au moins égale à 80% de la hauteur que présente la partie frontale de la monture au niveau des zones de jonction entre cette partie frontale et les éléments de protection latérale.

Comme usuel dans le domaine des montures de lunettes, la monture comprend avantageusement de plus un élément d'appui sur le nez d'un utilisateur en condition de port, lequel est, de préférence, un nez-selle. Ce nez-selle peut être soit monobloc avec la partie frontale de la monture, auquel cas il est constitué du même matériau radio-atténuateur que celui formant cette partie frontale et les éléments de protection latérale de la monture, soit rapporté sur la partie frontale de la monture, auquel cas il est préférentiellement en un matériau élastomère souple tel qu'un silicone pour plus de confort, ce matériau élastomère souple pouvant comprendre une charge radio-atténuatrice, de préférence identique à celle présente dans la partie frontale et les éléments de protection latérale de la monture.

L'invention a aussi pour objet des lunettes de protection contre les rayonnements ionisants, qui comprennent un ou deux verres optiques radio-atténuateurs enchâssés dans une monture et qui sont caractérisées en ce que la monture est telle que précédemment définie.

Conformément à l'invention, le ou les verres optiques radio-atténuateurs peuvent être des verres qui n'assurent aucune correction visuelle ou, au contraire, des verres qui corrigent un défaut visuel tel qu'une myopie, une hypermétropie, un astigmatisme isolé ou associé à une myopie ou une hypermétropie, ou bien une presbytie isolée ou associée à une myopie, une hypermétropie et/ou un astigmatisme, auquel cas ils peuvent être à simple foyer, à double foyer ou progressifs.

De préférence, ce ou ces verres sont des verres plombés, en particulier des verres minéraux chargés en plomb, typiquement sous la forme d'oxyde(s). Ils peuvent présenter une équivalence plomb de 0,50 mm, voire de 0,75 mm. De tels verres sont notamment disponibles auprès de la société SCHOTT et de la société HOYA.

La présence d'une charge radio-atténuatrice dans la partie frontale et les retours latéraux de la monture des lunettes de radioprotection de l'invention, voire dans les branches de ces lunettes si celles-ci comportent des branches, a pour effet d'augmenter le poids de ces lunettes par rapport à ce que ce poids serait si la monture était exempte de charge radio-atténuatrice.

De ce fait, pour que cette augmentation de poids ne se traduise pas par une perte d'ergonomie, il est prévu selon l'invention de munir optionnellement la monture des lunettes d'un premier élément d'attache qui est configuré pour s'attacher à un deuxième élément d'attache que comprend un dispositif de reprise du poids des lunettes lorsque les lunettes et le dispositif de reprise de poids sont portés par un utilisateur, les lunettes étant portées sur le visage de l'utilisateur et le dispositif de reprise de poids étant porté sur le crâne de l'utilisateur.

Conformément à l'invention, le premier élément d'attache est avantageusement un aimant qui est rapporté sur la monture ou intégré dans cette monture.

L'invention a encore pour objet un ensemble comprenant des lunettes de radioprotection telles que précédemment définies, et un dispositif de reprise de poids, dans lequel les lunettes comprennent un premier élément d'attache, le dispositif de reprise de poids comprend un deuxième élément d'attache et dans lequel les premier et deuxième éléments d'attache sont configurés pour s'attacher l'un à l'autre lorsque les lunettes et le dispositif de reprise de poids sont portés par un utilisateur, les lunettes étant portées sur le visage de l'utilisateur et le dispositif de reprise de poids étant porté sur le crâne de l'utilisateur.

Conformément à l'invention, le dispositif de reprise de poids peut être du type couvre-chef, c'est-à-dire configuré pour couvrir le sommet du crâne de l'utilisateur en condition de port, auquel cas il peut être une casquette, un bonnet ou analogue, ou bien être du type serre-tête, c'est-à-dire configuré pour n'entourer que la partie frontale du crâne.

De préférence, les premier et deuxième éléments d'attache sont des aimants de polarité opposée.

L'invention présente de nombreux avantages.

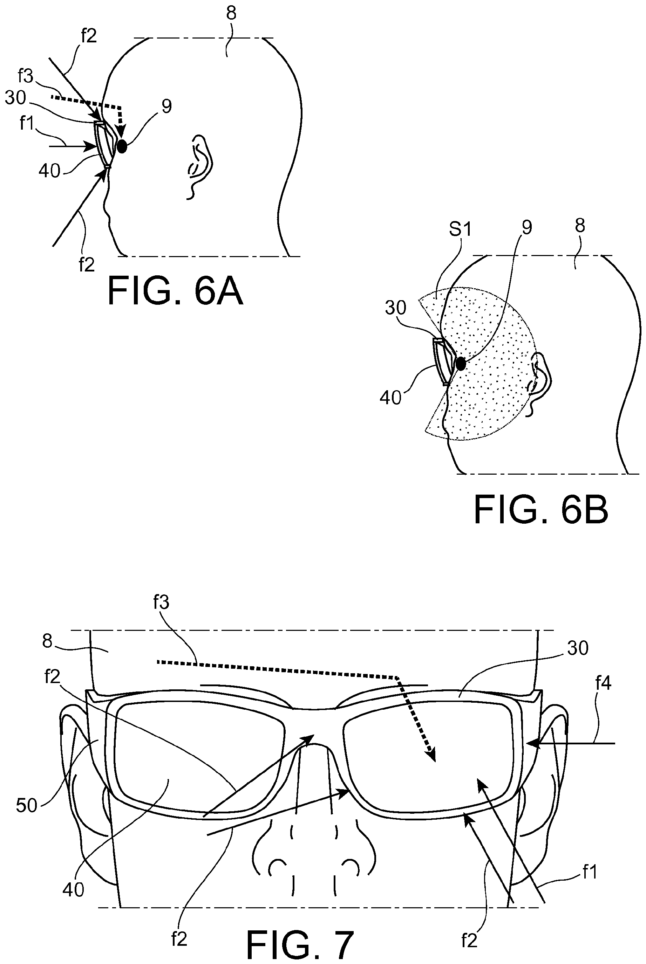

En effet, comme montré sur les figures 6A, 6B, 7, 8A et 8B, les lunettes de radioprotection de l'invention offrent, de par leur monture, une surface de protection contre les rayonnements ionisants considérablement augmentée par rapport à celle fournie par les lunettes de radioprotection de l'état de la technique avec, à la clé, une meilleure protection angulaire tant dans les plans verticaux médian et paramédians du cristallin que dans les plans horizontaux médian et paramédians du cristallin.

L'équivalent plomb que présentent actuellement les verres de lunettes de radioprotection de l'état de la technique et qui est typiquement de 0,75 mm a été choisi pour respecter un niveau dosimétrique donné, lequel est issu de l'énergie déposée par les rayonnements ionisants directs et indirects sur le cristallin. Les lunettes de radioprotection de l'invention fournissant une surface de protection accrue avec une augmentation du nombre de rayonnements directs atténués et une diminution de la contribution des rayonnements indirects, elles permettent d'envisager un abaissement de l'équivalent plomb nécessaire pour les verres et, partant, une diminution du poids de ces verres et, donc, du poids de l'ensemble des lunettes, à niveau dosimétrique équivalent.

En outre, les lunettes de radioprotection de l'invention permettent d'offrir une protection améliorée par rapport à celle fournie par les lunettes de radioprotection de l'état de la technique sans pour autant sacrifier à une ergonomie et à une esthétique propres à favoriser leur utilisation systématique par des travailleurs qui sont soumis à une exposition à des rayonnements ionisants.

De par leurs avantages, les lunettes de l'invention sont particulièrement adaptées à être utilisées dans l'industrie nucléaire, notamment pour la manipulation de poudres de combustibles nucléaires du type MOX.

Toutefois, il est également possible d'utiliser ces lunettes dans tous les autres secteurs professionnels dans lesquels une protection contre les rayonnements ionisants est recherchée et, en particulier, dans le secteur médical pour la radiologie diagnostique, la radiologie interventionnelle, la radiothérapie, la curiethérapie, la médecine nucléaire, etc.

D'autres caractéristiques et avantages de l'invention ressortiront du complément de description qui suit.

Il va de soi que ce complément de description n'est donné qu'à titre d'illustration de l'objet de l'invention et ne doit en aucun cas être interprété comme une limitation de cet objet.

### BRÈVE DESCRIPTION DES FIGURES

Les figures 1A et 1B illustrent deux exemples de lunettes de radioprotection de l'état de la technique, à protections frontale et latérale.
Les figures 2A et 2B représentent schématiquement et partiellement la tête d'une personne, vue de profil gauche, portant des lunettes de radioprotection telles qu'illustrées sur la figure 1A.
La figure 3 représente schématiquement et partiellement la tête d'une personne, vue de face, portant des lunettes de radioprotection telles qu'illustrées sur la figure 1A.
Les figures 4A et 4B représentent schématiquement et partiellement la tête d'une personne, vue de dessus, portant des lunettes de radioprotection telles qu'illustrées sur la figure 1A.
Les figures 5A, 5B, 5C et 5D illustrent quatre exemples de lunettes de radioprotection de l'invention.
Les figures 6A et 6B sont des représentations analogues à celles des figures 2A et 2B mais pour une personne portant des lunettes de radioprotection telles qu'illustrées sur la figure 5A.
La figure 7 est une représentation analogue à celle de la figure 3 mais pour une personne portant des lunettes de radioprotection telles qu'illustrées sur la figure 5A.
Les figures 8A et 8B sont des représentations analogues à celles des figures 4A et 4b mais pour une personne portant des lunettes de radioprotection telles qu'illustrées sur la figure 5A.
La figure 9A représente schématiquement la tête d'une personne, vue de face, portant un exemple d'un ensemble lunettes de radioprotection/dispositif de reprise de poids de l'invention, tandis que la figure 9B représente schématiquement et partiellement la tête de cette personne mais vue de profil gauche.

### EXPOSÉ DÉTAILLÉ DE MODES DE RÉALISATION PARTICULIERS

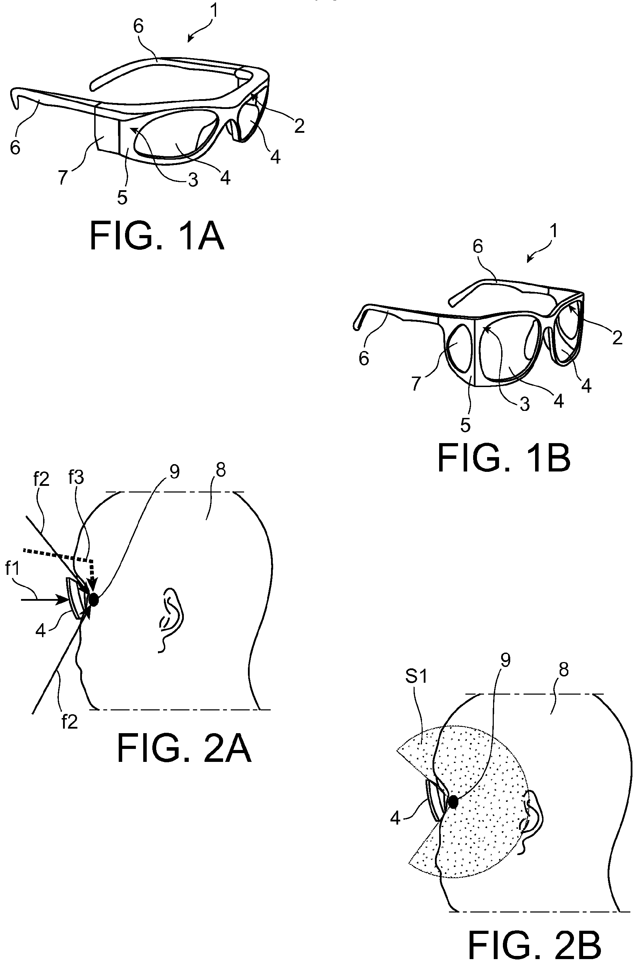

On se réfère tout d'abord aux figures 1A et 1B qui illustrent deux exemples de lunettes de radioprotection de l'état de la technique, à protections frontale et latérale.

Ces lunettes, qui sont référencées 1, comprennent une monture 2 qui, elle-même, comprend une partie frontale 3, ou face, dans laquelle sont enchâssés deux verres optiques 4 et qui est prolongée latéralement par deux retours 5, monoblocs avec la face, et deux branches 6 articulées sur les extrémités opposées des retours latéraux 5.

La monture 2 est en un matériau plastique transparent aux rayonnements ionisants et la protection frontale est uniquement assurée par les verres 4 qui sont des verres plombés.

Dans l'exemple montré sur la figure 1A, la protection latérale est assurée par deux plaquettes 7 de plomb de forme sensiblement rectangulaire, qui sont rapportées sur une partie des retours latéraux 5, à distance des bords externes des verres plombés 4.

Dans l'exemple montré sur la figure 1B, la protection latérale est assurée par deux pièces 7 en verre plombé de forme sensiblement triangulaire, qui sont intégrées dans une partie des retours latéraux 5, également à distance des bords externes des verres plombés 4.

Dans les deux cas, les éléments de protection latérale ont typiquement une équivalence plomb de 0,35 mm.

On se réfère à présent aux figures 2A et 2B qui représentent schématiquement et partiellement la tête 8 d'une personne, vue de profil gauche, portant des lunettes de radioprotection telles qu'illustrées sur la figure 1A, ainsi que le cristallin 9 de cette personne.

Sur les figures 2A et 2B, qui sont destinées à montrer les limites de la protection frontale qui est assurée par ces lunettes, le verre plombé 4 de gauche est représenté, vu en coupe transversale. Par contre, ne sont représentées ni la face 3 de la monture puisque celle-ci est en un matériau transparent aux rayonnements ionisants ni la plaquette 7 de gauche pour des raisons de lisibilité.

Comme visible sur la figure 2A, la protection frontale se limite aux rayonnements « directs » qui frappent les verres plombés et dont un exemple est matérialisé par la flèche f1.

Ainsi, le cristallin 9 n'est protégé :
- ni des rayonnements « directs » qui frappent les parties supérieure et inférieure de la face de la monture et dont deux exemples sont matérialisés par les flèches f2,
- ni des rayonnements « diffusés » qui sont issus d'une interaction de rayonnements « directs » avec les zones cutanées entourant les lunettes et dont un exemple est matérialisé par la flèche f3.

Il en résulte que le secteur angulaire non radioprotégé dans le plan vertical médian du cristallin 9 correspond au secteur noté S1 sur la figure 2B.

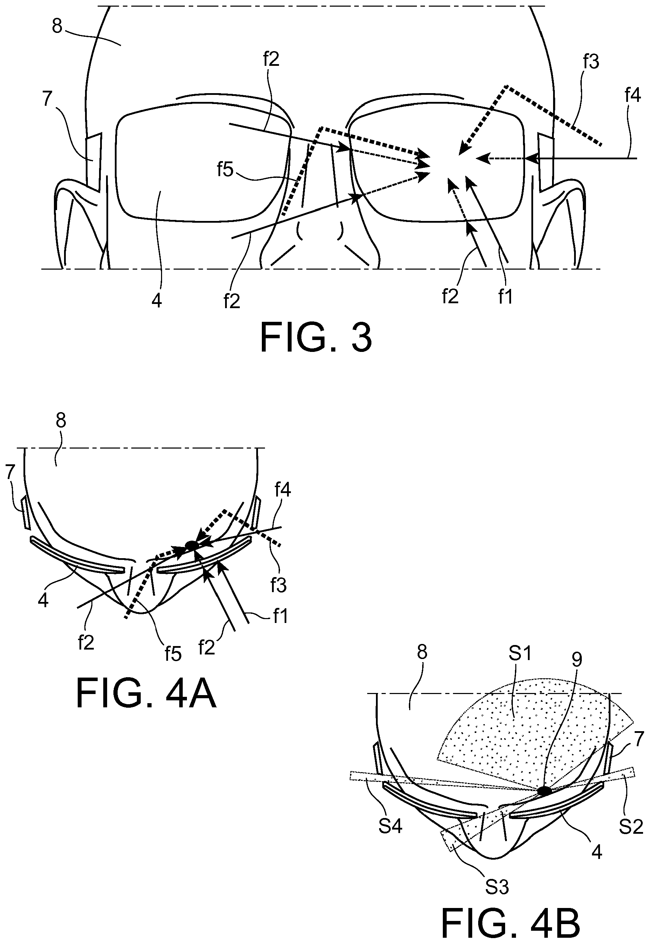

On se réfère à présent à la figure 3 qui représente schématiquement et partiellement la tête 8 montrée sur les figures 2A et 2B mais vue de face.

Sur la figure 3, qui est destinée à montrer les limites des protections frontale et latérale qui sont assurées par des lunettes de radioprotection telles qu'illustrées sur la figure 1A, sont visibles les verres plombés 4 et les plaquettes 7 de ces lunettes. Par contre, n'est pas représentée leur monture pour les mêmes raisons que précédemment.

Là également, la figure 3 montre que ces lunettes protègent le cristallin des rayonnements « directs » qui frappent les verres plombés et dont un exemple est matérialisé par la flèche f1 mais qu'elles ne le protègent :
- ni des rayonnements « directs » qui frappent la face de la monture et dont trois exemples sont matérialisés par les flèches f2,
- ni des rayonnements « diffusés » qui sont issus d'une interaction de rayonnements « directs » avec les zones cutanées entourant les lunettes et dont un exemple est matérialisé par la flèche f3.

Elle montre aussi que ces lunettes ne protègent pas non plus le cristallin :
- des rayonnements « directs » qui frappent les parties des retours latéraux qui sont situées entre les extrémités latérales de la face de la monture et les plaquettes de plomb et dont un exemple est matérialisé par la flèche f4, et
- des rayonnements « diffusés » qui sont issus de l'interaction de rayonnements directs avec les zones cutanées telles que la racine du nez et la partie supérieure du dos du nez qui, bien que couvertes par la monture, ne sont pas protégées par elle et dont un exemple est matérialisé par la flèche f5.

Une autre illustration des limites des protections frontale et latérale fournies par des lunettes de radioprotection telles qu'illustrées sur la figure 1A est donnée sur les figures 4A et 4B qui représentent schématiquement et partiellement la tête 8 montrée sur les figures 2A, 2B et 3 mais vue de dessus.

La figure 4A reprend les différents types de rayonnements matérialisés par les flèches f1 à f5 sur la figure 3 tandis que la figure 4B montre les 4 secteurs angulaires non radioprotégés dans le plan horizontal médian du cristallin 9, respectivement notés S2, S3, S4 et S5, avec ces lunettes.

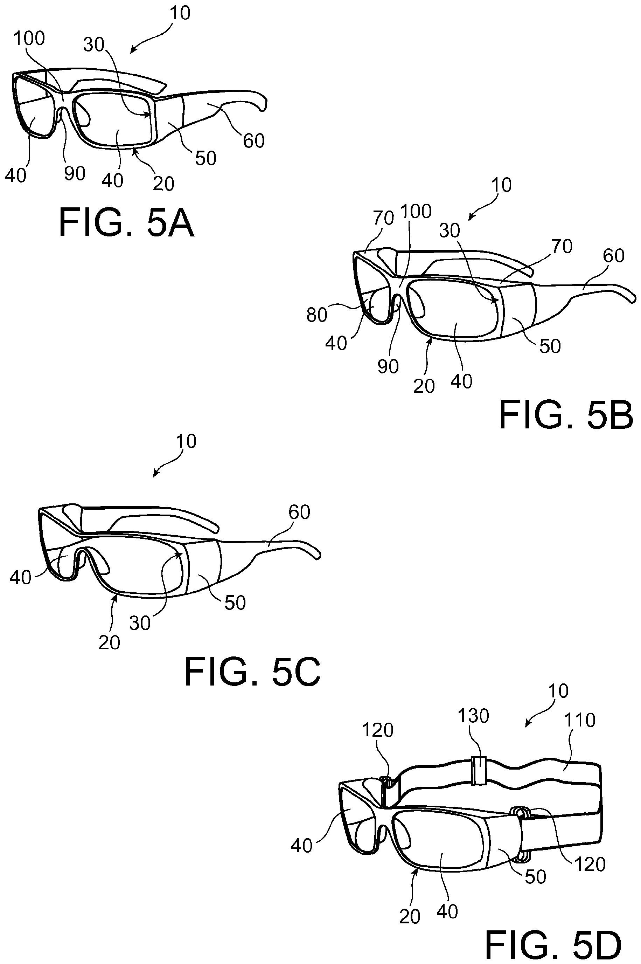

On se réfère à présent aux figures 5A et 5B qui illustrent deux premiers exemples de lunettes de radioprotection de l'invention.

Ces lunettes, qui sont référencées 10, comprennent une monture 20 qui, elle-même, comprend une partie frontale 30, ou face, dans laquelle sont enchâssés deux verres optiques 40 et qui est prolongée latéralement par deux retours 50, monoblocs avec la face, et deux branches 60 articulées - *via* deux charnières (non représentées) - sur les extrémités opposées des retours latéraux 50.

Comme dans les lunettes illustrées sur les figures 1A et 1B, les verres 40 sont des verres plombés.

Par contre, à la différence des lunettes illustrées sur les figures 1A et 1B, la face 30 de la monture ainsi que les retours latéraux 50 sont en un matériau radio-atténuateur qui comprend une matrice polymère, par exemple en acétopropionate de cellulose, en polyamide ou copolyamide, en résine époxyde ou en polyuréthanne, dans laquelle est dispersée une charge radio-atténuatrice comprenant, par exemple, des particules d'un oxyde de plomb, d'erbium, d'un oxyde d'erbium, de praséodyme, d'un oxyde de praséodyme, d'un mélange d'un oxyde d'erbium et d'un oxyde de praséodyme, d'un mélange d'un oxyde d'erbium, d'un oxyde de praséodyme et de bismuth, ou d'un mélange d'un oxyde de bismuth, d'un oxyde de tungstène et d'un oxyde de lanthane, ou composée de telles particules.

Dans l'exemple illustré sur la figure 5A, la monture 20 est du type classiquement dénommé « rectangulaire » dans le domaine des montures optiques, c'est-à-dire avec une face 30 de forme sensiblement rectangulaire et présentant un faible angle de cintre (inférieur à 10°) et des retours latéraux 50 qui s'étendent sensiblement perpendiculairement à la face 30. La hauteur de ces retours latéraux est sensiblement équivalente à celle que présente la face 30 au niveau de la jonction desdits retours latéraux sur ladite face.

Dans l'exemple illustré sur la figure 5B, la monture 20 est une monture plus enveloppante que celle des lunettes illustrées sur la figure 5A, avec une face 30 qui présente un angle de cintre plus prononcé et la présence de deux éléments 70 de protection sus-orbitaire et de deux éléments 80 de protection sub-orbitaires qui sont monoblocs avec la face 30 et les retours latéraux 50.

Dans l'exemple illustré sur la figure 5A comme dans l'exemple illustré sur la figure 5B, la monture 20 comprend de plus un nez-selle 90, lequel peut être rapporté à la base du pont de nez 100 réunissant les deux verres optiques 40, auquel cas il est avantageusement en un matériau élastomère souple tel qu'un silicone, renfermant éventuellement une charge radio-atténuatrice, ou bien être intégré au pont de nez 100, c'est-à-dire monobloc avec ce pont de nez.

La figure 5C illustre un troisième exemple de lunettes 10 de radioprotection de l'invention. Ces lunettes ne diffèrent de celles montrées sur la figure 5B qu'en ce que la monture 20 est « mono-écran », c'est-à-dire avec une face 30 dans laquelle n'est enchâssé qu'un seul verre optique 40 qui s'étend d'un bord latéral à l'autre de ladite face et qui couvre, donc, les deux zones orbitaires d'un utilisateur en condition de port.

La figure 5D illustre un quatrième exemple de lunettes 10 de radioprotection de l'invention qui ne diffèrent de celles montrées sur la figure 5B qu'en ce que la monture 20 comprend un bandeau de maintien 110 au lieu de branches.

Ce bandeau de maintien, qui est préférentiellement en une matière élastique, par exemple un textile élastique ou un élastomère tel que le néoprène, est raccordé aux extrémités opposées des retours latéraux 50 de la monture 20, par exemple *via* deux passants 120 qui sont monoblocs avec ces retours latéraux et au niveau desquels les extrémités du bandeau forment une boucle fermée par un élément de fixation (non représenté) de type bouton-pression, rivet, adhésif repositionnable, bande auto-agrippante du type velours-crochets (ou Velcro^{™}) ou analogue. Le bandeau de maintien est, par ailleurs, muni d'un élément 130 permettant de l'ouvrir, de le fermer et/ou d'en ajuster la taille à la circonférence de la tête d'un utilisateur.

On se réfère à présent aux figures 6A et 6B qui sont des représentations analogues à celles des figures 2A et 2B mais pour une personne portant des lunettes de radioprotection telles qu'illustrées sur la figure 5A. De ce fait, est représentée sur ces figures la face 30 de la monture 20, vue en coupe transversale.

Comme le montre la figure 6A, les lunettes de l'invention permettent de protéger le cristallin 9 non seulement des rayonnements « directs » qui frappent les verres plombés et dont un exemple est matérialisé par la flèche f1, mais également des rayonnements « directs » qui frappent les parties supérieure et inférieure de la face de la monture et dont deux exemples sont matérialisés par les flèches f2.

De ce fait, comme visible sur la figure 6B, le secteur angulaire non radioprotégé dans le plan vertical médian du cristallin 9, noté S1, obtenu avec les lunettes de l'invention, est notablement diminué par rapport à celui montré sur la figure 2B.

La figure 7 est également une représentation analogue à celle de la figure 3 mais pour une personne portant des lunettes de radioprotection telles qu'illustrées sur la figure 5A.

Cette figure montre que ces lunettes protègent le cristallin à la fois :
- des rayonnements « directs » qui frappent les verres plombés et dont un exemple est matérialisé par la flèche f1,
- des rayonnements « directs » qui frappent la face de la monture et dont trois exemples sont matérialisés par les flèches f2, et
- des rayonnements « directs » qui frappent les retours latéraux et dont un exemple est matérialisé par la flèche f4.

Par voie de conséquence, sont aussi supprimés les rayonnements « diffusés » issus de l'interaction de rayonnements directs avec les zones cutanées couvertes par la monture tels que celui matérialisé sur la figure 3 par la flèche f5.

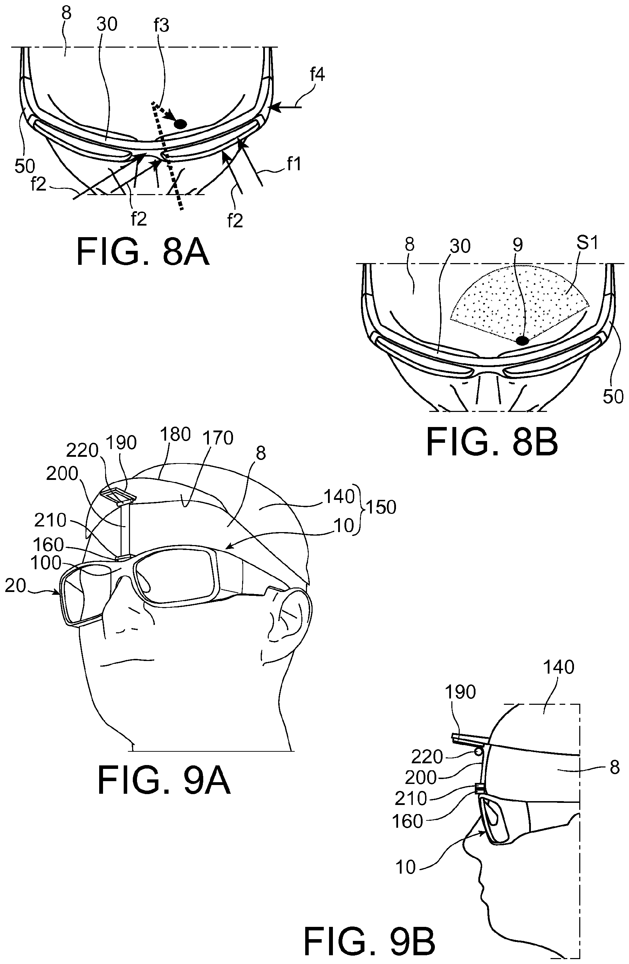

Ceci est aussi montré par la figure 8A qui est une représentation analogue à celle de la figure 4A mais pour une personne portant des lunettes de radioprotection telles qu'illustrées sur la figure 5A et sur laquelle ont été repris les différents types de rayonnements matérialisés par les flèches f1 à f5 sur la figure 7.

De ce fait, comme visible sur la figure 8B qui est à comparer à la figure 4B puisqu'il s'agit d'une représentation analogue à celle de la figure 4B mais pour une personne portant des lunettes de radioprotection telles qu'illustrées sur la figure 5A, il ne reste plus, avec les lunettes de radioprotection de l'invention, qu'un seul secteur angulaire non radioprotégé dans le plan horizontal médian du cristallin 9, à savoir le secteur S1, lequel est, toutefois, notablement réduit par rapport au secteur angulaire S1 visible sur la figure 4B.

À titre d'exemple, des lunettes de radioprotection telles qu'illustrées sur la figure 5A ont été réalisées avec des verres plombés SCHOTT SF6, une monture et des retours latéraux de 5 mm d'épaisseur, comprenant du sesquioxyde de bismuth Bi₂O₃ dispersé dans une matrice de polyuréthanne à hauteur de 50 % massiques pour obtenir une épaisseur plomb de 0,50 mm. Ces lunettes pèsent 92 g.

Aussi, pour ne pas perdre en ergonomie, l'invention propose-t-elle de porter ces lunettes conjointement avec un dispositif 140 de reprise de poids comme illustré sur les figures 9A et 9B qui représentent schématiquement la tête 8 d'une personne, vue respectivement de face (figure 9A) et de profil (figure 9B), portant un exemple d'un ensemble lunettes 10 de radioprotection/dispositif 140 de reprise de poids de l'invention.

Comme visible sur ces figures, dans cet ensemble, qui est référencé 150, la monture 20 des lunettes, qui sont sensiblement du même type que celles illustrées sur la figure 5A, est munie d'un aimant 160 qui est rapporté sur le bord supérieur du pont de nez 100 mais qui pourrait tout aussi bien être intégré dans ce pont de nez.

Le dispositif 140 de reprise de poids se présente, lui, sous la forme d'une casquette.

La partie inférieure 170 de la visière 180 de cette casquette est munie d'un organe 190 de maintien d'une languette 200 dont l'extrémité inférieure comprend un aimant 210 similaire en taille à celui que comprend la monture 20 des lunettes mais de polarité opposée et dont l'extrémité supérieure est fixée de façon amovible sur cet organe, par exemple par clipsage, par un adhésif repositionnable ou par une bande auto-agrippante du type Velcro^{™}. Dans ce dernier cas, la languette peut, elle-même, être une bande auto-agrippante.

Une pièce cylindrique 220, solidaire de l'organe 190 de maintien, permet de maintenir toute la partie de la languette qui est située entre cette pièce et l'aimant 210 à l'aplomb du pont de nez 100 des lunettes.

La fixation amovible de l'extrémité supérieure de la languette 200 sur l'organe 190 de maintien permet, elle, d'ajuster la longueur de la partie de la languette 200 qui est comprise entre la pièce 220 et l'aimant 210 à la hauteur du front d'un utilisateur qui est comprise entre la visière 180 et la racine du nez de cet utilisateur en fonction des conditions de port de la casquette par celui-ci.

Une fois cet ajustement réalisé, la monture 20 des lunettes est automatiquement solidarisée à la casquette par l'effet magnétique qui s'établit entre les aimants 160 et 210 et une partie du poids exercé par la monture 20 sur la racine nasale de l'utilisateur est automatiquement repris par cette casquette.

## Revendications

1. Monture (20) de lunettes de protection contre les rayonnements ionisants, comprenant une partie frontale (30) se prolongeant latéralement par deux éléments (50) de protection latérale, et un dispositif (60, 110) de maintien de la monture sur le visage d'un utilisateur en condition de port,
les éléments de protection latérale sont monoblocs avec la partie frontale,
la partie frontale et les éléments de protection latérale sont en un matériau radio-atténuateur comprenant une matrice polymère dans laquelle est dispersée une charge radio-atténuatrice, **caractérisée en ce que**
la charge radio-atténuatrice comprend des particules d'au moins un métal choisi parmi les terres rares, le bismuth, l'antimoine, l'étain, le baryum, le tantale, ou d'au moins un composé choisi parmi les alliages et les oxydes de plomb, de terres rares, de bismuth, d'antimoine, d'étain, de tungstène, de baryum, de tantale.

2. Monture de lunettes selon la revendication 1, dans laquelle la charge radio-atténuatrice comprend des particules d'un oxyde de plomb, d'erbium, d'un oxyde d'erbium, de praséodyme, d'un oxyde de praséodyme, d'un mélange d'un oxyde d'erbium et d'un oxyde de praséodyme, d'un mélange d'un oxyde d'erbium, d'un oxyde de praséodyme et d'un oxyde de bismuth, ou d'un mélange d'un oxyde de bismuth, d'un oxyde de tungstène et d'un oxyde de lanthane.

3. Monture de lunettes selon la revendication 1 ou la revendication 2, dans laquelle la charge radio-atténuatrice comprend des particules de bismuth ou d'un oxyde de bismuth.

4. Monture de lunettes selon l'une quelconque des revendications 1 à 3, dans laquelle le polymère de la matrice est choisi parmi les acétates de cellulose, les acétopropionates de cellulose, les polyamides et copolyamides, les résines époxydes et les polyuréthannes.

5. Monture de lunettes selon l'une quelconque des revendications 1 à 4, dans laquelle le matériau radio-atténuateur comprend de 20% à 95% en masse de charge radio-atténuatrice.

6. Monture de lunettes selon l'une quelconque des revendications 1 à 5, dans laquelle la partie frontale (30) est configurée pour enchâsser un ou deux verres optiques (40).

7. Monture de lunettes selon l'une quelconque des revendications 1 à 6, qui comprend de plus :
deux éléments (70) de protection sus-orbitaire monoblocs avec la partie frontale (30) et les éléments (50) de protection latérale et/ou
deux éléments (80) de protection sub-orbitaire monoblocs avec la partie frontale (30) et les éléments (50) de protection latérale.

8. Monture de lunettes selon l'une quelconque des revendications 1 à 7, dans laquelle le dispositif de maintien de la monture comprend :
deux branches (60) articulées sur les éléments (50) de protection latérale ou un bandeau (110) raccordé aux éléments (50) de protection latérale et/ou
un nez-selle (90) monobloc avec la partie frontale (30) ou rapporté sur la partie frontale (30).

9. Lunettes (10) de protection contre les rayonnements ionisants, comprenant un ou deux verres optiques radio-atténuateurs (40) enchâssés dans une monture (20) selon l'une quelconque des revendications 1 à 8, le(s) verre(s) optique(s) radio-atténuateur(s) (40) étant, de préférence, un (des) verre(s) plombé(s).

10. Lunettes selon la revendication 9, dans lesquelles la monture (20) comprend un premier (160) élément d'attache qui est configuré pour s'attacher à un deuxième (210) élément d'attache que comprend un dispositif (140) de reprise de poids des lunettes lorsque les lunettes et le dispositif de reprise de poids sont portés par un utilisateur, les lunettes étant portées sur le visage de l'utilisateur et le dispositif de reprise de poids étant porté sur le crâne de l'utilisateur.

11. Lunettes selon la revendication 10, dans lesquelles le premier (160) élément d'attache est un aimant rapporté sur la monture (20) ou intégré dans la monture (20).

12. Ensemble (150) comprenant des lunettes (10) de radioprotection selon l'une quelconque des revendications 9 à 11, et un dispositif (140) de reprise de poids, dans lequel les lunettes comprennent un premier (160) élément d'attache, le dispositif de reprise de poids comprend un deuxième (210) élément d'attache et dans lequel les premier et deuxième éléments d'attache sont configurés pour s'attacher l'un à l'autre lorsque les lunettes et le dispositif de reprise de poids sont portés par un utilisateur, les lunettes étant portées sur le visage de l'utilisateur et le dispositif de reprise de poids étant porté sur le crâne de l'utilisateur.

13. Ensemble selon la revendication 12, dans lequel les premier (160) et deuxième (210) éléments d'attache sont des aimants de polarité opposée.

14. Ensemble selon la revendication 12 ou la revendication 13, dans lequel le dispositif (140) de reprise de poids est configuré pour couvrir le sommet du crâne de l'utilisateur ou pour n'entourer que la partie frontale du crâne de l'utilisateur en condition de port.

## Patentansprüche

1. Gestell (20) für eine Brille zum Schutz vor ionisierenden Strahlungen, umfassend einen vorderen Teil (30), der seitlich durch zwei Elemente (50) zum seitlichen Schutz verlängert wird, und eine Vorrichtung (60, 110) zum Halten des Gestells auf dem Gesicht eines Benutzers im Zustand des Tragens,
die Elemente zum seitlichen Schutz sind mit dem vorderen Teil einstückig,
der vordere Teil und die Elemente zum seitlichen Schutz bestehen aus einem strahlungsdämpfenden Material, das eine Polymermatrix umfasst, in der eine strahlungsdämpfende Ladung dispergiert ist, **dadurch gekennzeichnet, dass**
die strahlungsdämpfende Ladung Partikel von mindestens einem Metall umfasst, das aus seltenen Erden, Wismut, Antimon, Zinn, Barium und Tantal ausgewählt ist, oder aus mindestens einer Verbindung, die aus den Legierungen und den Oxiden von Blei, seltenen Erden, Wismut, Antimon, Zinn, Wolfram, Barium und Tantal ausgewählt ist.

2. Gestell für eine Brille nach Anspruch 1, wobei die strahlungsdämpfende Ladung Partikel von Bleioxid, Erbium, einem Erbiumoxid, Praseodym, einem Praseodymoxid, einer Mischung aus einem Erbiumoxid und einem Praseodymoxid, einer Mischung aus einem Erbiumoxid, einem Praseodymoxid und einem Wismutoxid oder aus einer Mischung aus einem Wismutoxid, einem Wolframoxid und einem Lanthanoxid umfasst.

3. Gestell für eine Brille nach Anspruch 1 oder Anspruch 2, wobei die strahlungsdämpfende Ladung Partikel von Wismut oder einem Wismutoxid umfasst.

4. Gestell für eine Brille nach einem der Ansprüche 1 bis 3, wobei das Polymer der Matrix aus Celluloseacetaten, Celluloseacetopropionaten, Polyamiden und Copolyamiden, Epoxidharzen und Polyurethanen ausgewählt ist.

5. Gestell für eine Brille nach einem der Ansprüche 1 bis 4, wobei das strahlungdämpfende Material zwischen 20 Gew.-% und 95 Gew.-% strahlungsdämpfende Ladung umfasst.

6. Gestell für eine Brille nach einem der Ansprüche 1 bis 5, wobei der vordere Teil (30) konfiguriert ist, um ein oder zwei optische Gläser (40) einzubetten.

7. Gestell für eine Brille nach einem der Ansprüche 1 bis 6, das außerdem Folgendes umfasst:
zwei Elemente (70) zum supraorbitalen Schutz, einstückig mit dem vorderen Teil (30) und den Elementen (50) zum seitlichen Schutz und/oder
zwei Elemente (80) zum suborbitalen Schutz, einstückig mit dem vorderen Teil (30) und den Elementen (50) zum seitlichen Schutz.

8. Gestell für Brille nach einem der Ansprüche 1 bis 7, wobei die Vorrichtung zum Halten des Gestells Folgendes umfasst:
zwei Schenkel (60), die an den Elementen (50) zum seitlichen Schutz angelenkt sind, oder eine Blende (110), die mit den Elementen (50) zum seitlichen Schutz verbunden ist, und/oder
eine Sattelnase (90), die mit dem vorderen Teil (30) einstückig ist oder auf dem vorderen Teil (30) aufgesetzt ist.

9. Brille (10) zum Schutz gegen ionisierende Strahlung, die ein oder zwei strahlungsdämpfende optische Gläser (40) umfasst, eingebettet in ein Gestell (20) nach einem der Ansprüche 1 bis 8, wobei das/die strahlungsdämpfende(n) optische(n) Glas (Gläser) (40) vorzugsweise ein (mehrere) verplombte(s) Glas (Gläser) ist (sind).

10. Brille nach Anspruch 9, wobei das Gestell (20) ein erstes (160) Befestigungselement umfasst, das konfiguriert ist, um an einem zweiten (210) Befestigungselement befestigt zu werden, das eine Vorrichtung (140) zur Wiederaufnahme des Gewichts der Brille umfasst, während die Brille und die Vorrichtung zur Wiederaufnahme des Gewichts von einem Benutzer getragen werden, wobei die Brille auf dem Gesicht des Benutzers getragen wird und wobei die Vorrichtung zur Wiederaufnahme des Gewichts auf dem Schädel des Benutzers getragen wird.

11. Brille nach Anspruch 10, wobei das erste (160) Befestigungselement ein Magnet ist, der an dem Gestell (20) angebracht oder in das Gestell (20) integriert ist.

12. Set (150), das eine Brille (10) zum Strahlenschutz nach einem der Ansprüche 9 bis 11 und eine Vorrichtung (140) zur Wiederaufnahme des Gewichts umfasst, wobei die Brille ein erstes (160) Befestigungselement umfasst, wobei die Vorrichtung zur Wiederaufnahme des Gewichts ein zweites (210) Befestigungselement umfasst und wobei das erste und das zweite Befestigungselement konfiguriert sind, um aneinander befestigt zu werden, während die Brille und die Vorrichtung zur Wiederaufnahme des Gewichts von einem Benutzer getragen werden, wobei die Brille auf dem Gesicht des Benutzers getragen wird und die Vorrichtung zur Wiederaufnahme des Gewichts auf dem Schädel des Benutzers getragen wird.

13. Set nach Anspruch 12, wobei das erste (160) und das zweite (210) Befestigungselement Magnete von entgegengesetzter Polarität sind.

14. Set nach Anspruch 12 oder Anspruch 13, wobei die Vorrichtung (140) zur Wiederaufnahme des Gewichts konfiguriert ist, um im Zustand des Tragens den Scheitelpunkt des Schädels des Benutzers zu bedecken oder um nur die Vorderseite des Schädels des Benutzers zu umschließen.

## Claims

1. Frame (20) for protective eyewear against ionising radiation, comprising a frontal part (30) being laterally extended by two side protective elements (50), and a device (60, 110) for holding the frame on a user's face in wearing condition,
the side protective elements are as a single piece with the frontal part,
the frontal part and the side protective elements are made of a radiation attenuating material comprising a polymer matrix in which a radiation attenuating load is dispersed, **characterised in that**
the radiation attenuating load comprises particles of at least one metal selected from rare earths, bismuth, antimony, tin, barium, tantalum, or of at least one compound selected from alloys and oxides of lead, rare earths, bismuth, antimony, tin, tungsten, barium, tantalum.

2. Frame for eyewear according to claim 2, wherein the radiation attenuating load comprises particles of a lead oxide, erbium, an erbium oxide, praseodymium, a praseodymium oxide, a mixture of an erbium oxide and a praseodymium oxide, a mixture of an erbium oxide, a praseodymium oxide and a bismuth oxide, or a mixture of a bismuth oxide, a tungsten oxide and a lanthanum oxide.

3. Frame for eyewear according to claim 1 or claim 2, wherein the radiation attenuating load comprises particles of bismuth or a bismuth oxide.

4. Frame for eyewear according to any of claims 1 to 3, wherein the polymer of the matrix is selected from cellulose acetates, cellulose acetopropionates, polyamides and copolyamides, epoxide resins and polyurethanes.

5. Frame for eyewear according to any of claims 1 to 4, wherein the radiation attenuating material comprises from 20% to 95% by mass of radiation attenuating load.

6. Frame for eyewear according to any of claims 1 to 5, wherein the frontal part (30) is configured to embed one or two optical glasses (40).

7. Frame for eyewear according to any of claims 1 to 6, which further comprises:
two supraorbital protective elements (70) as a single piece with the frontal part (30) and the side protective elements (50) and/or
two infraorbital protective elements (80) as a single piece with the frontal part (30) and the side protective elements (50).

8. Frame for eyewear according to any of claims 1 to 7, wherein the frame holding device comprises:
two arms (60) hinged to the side protective elements (50) or a headband (110) connected to the side protective elements (50) and/or
a saddle bridge (90) as a single piece with the frontal part (30) or assembled to the frontal part (30).

9. Protective eyewear (10) against ionising radiation, comprising one or two radiation attenuating optical glasses (40) embedded in a frame (20) according to any of claims 1 to 8, the radiation attenuating optical glass(es) (40) being preferably lead glass(es).

10. Eyewear according to claim 9, wherein the frame (20) comprises a first attaching element (160) which is configured to attach to a second attaching element (210) comprised by a weight take-up device (140) for eyewear when the eyewear and the weight take-up device are worn by a user, the eyewear being worn on the user's face and the weight take-up device being worn on the user's skull.

11. Eyewear according to claim 10, wherein the first attaching element (160) is a magnet assembled to the frame (20) or integrated into the frame (20).

12. Assembly (150) comprising radiation protective eyewear (10) according to any of claims 9 to 11, and a weight take-up device (140), wherein the eyewear comprises a first attaching element (160), the weight take-up device comprises a second attaching element (210) and wherein the first and second attaching elements are configured to attach to each other when the eyewear and the weight take-up device are worn by a user, the eyewear being worn on the user's face and the weight take-up device being worn on the user's skull.

13. Assembly according to claim 12, wherein the first (160) and second (210) attaching elements are magnets with opposite polarity.

14. Assembly according to claim 12 or claim 13, wherein the weight take-up device (140) is configured to cover the top of the user's skull or to surround only the frontal part of the user's skull in wearing condition.
